# EUROPÄISCHE PATENTANMELDUNG

(11) **EP 1 440 792 A1**
(43) Veröffentlichungstag der Anmeldung: **28.07.2004**
(21) Anmeldenummer: 03029164.5
(22) Anmeldetag: 18.12.2003
(51) Int. Cl.: B32B 27/08, C09J 7/02

(54) **Verwendung eines Laminates**

(30) Priorität: 21.01.2003 DE 10302417
(71) Anmelder: Huhtamaki Forchheim Zweigniederlassung der Huhtamaki Deutschland GmbH & Co. KG, 91299 Forchheim (DE)
(72) Erfinder: Günter, Walter, 91301 Forchheim (DE); Müller, Josef, 91301 Forchheim (DE)
(74) Vertreter: Hutzelmann, Gerhard

(57) **Zusammenfassung**

Verwendung eines Laminates aus wenigstens drei Lagen, mit einer Träger-Lage, auf welcher ein Kleber angeordnet ist und einer Release-Folien-Lage, zum Ersatz eines Aufbaues aus einer sogenannten Backsheet-Folie, einem Kleber und einer Release-Abdeckbahn, wobei wenigstens ein Teil der Lagen coextrudiert ist.

## Beschreibung

Die Erfindung bezieht sich auf die Verwendung eines Laminates aus wenigstens drei Lagen, mit einer Träger-Lage, auf welcher ein Kleber angeordnet ist und einer Release-Folien-Lage, zum Ersatz eines Aufbaues aus einer sogenannten Backsheet-Folie, einem Kleber und einer Release-Abdeckbahn.

Insbesondere bei Damenbinden und dergleichen wird eine sogenannte Backsheet-Folie als Aussenlage verwendet, die zum Festlegen der Damenbinde an der Wäsche mit Kleber versehen ist. Bis zur Verwendung der Damenbinde muss diese Kleberschicht zusätzlich abgedeckt werden, da sie sonst ihre Klebkraft verliert. Für diese Abdeckung werden meist beschichtete Papiere eingesetzt. Die Herstellung eines derartigen Verbundes ist sehr aufwendig und damit teuer.

Der Erfindung liegt die Aufgabe zugrunde, die Verwendung eines einfacher herzustellenden Laminates vorzuschlagen.

Diese Aufgabe wird erfindungsgemäß dadurch gelöst, dass wenigstens ein Teil der Lagen coextrudiert ist.

Als besonders vorteilhaft hat es sich dabei ergeben, wenn ein Laminat verwendet wird, bei dem wenigstens die Release-Folien-Lage coextrudiert ist.

Eine weitere vorteilhafte Verwendung ergibt sich dadurch, dass der Kleber auf eine Träger-Lage aufextrudiert ist, wobei bei dieser Extrusion die ebenfalls coextrudierte Release-Folien-Lage zuläuft.

Eine weitere vorteilhafte Ausgestaltung liegt darin, dass die Träger-Lage für den Kleber aus einem Folien/Vlies-Verbund besteht.

Besonders vorteilhaft ist es auch, wenn ein Laminat verwendet wird, das dadurch gekennzeichnet ist, dass die Folie der Träger-Lage des Klebers einen hohen Anteil an Füllstoffes, wie z.B. CaC03 enthält.

Ebenfalls sehr günstig ist die Verwendung eines Laminat, bei dem wenigstens Teile des Laminates gereckt sind.

Ein weiteres Laminat für die erfindungsgemäße Verwendung ist dadurch gekennzeichnet, dass das Laminat wenigstens einseitig geprägt ist.

Ebenfalls sehr vorteilhaft für die erfindungsgemäße Verwendung ist es, wenn wenigstens die Träger-Lage für den Kleber eingefärbt ist.

Die erfindungsgemäße Verwendung eines wenigstens teilweise coextrudierten Laminates anstelle eines einzeln aufgebauten Verbundes hat eine Reihe von Vorteilen.

Die einzelnen Lagen können entsprechend den für die Verwendung vorgesehenen und notwendigen Eigenschaften hergestellt werden, da Lagen wenigstens teilweise coextrudiert sind und nicht separat hergestellt werden müssen. Durch ein eventuell durchgeführtes Recken des gesamten Laminates werden die Festigkeit und Wasserdampf-Diffusion verbessert. Auch die Prägung des gesamten Laminates wirkt sich sehr günstig aus, da ein Prägen nur der Träger-Lage des Klebers die Weiterverarbeitung zu einem Verbund stark erschweren würde. Andererseits kann aber durch die Prägung die Haptik des Laminates wesentlich verbessert werden.

## Patentansprüche

1. Verwendung eines Laminates aus wenigstens drei Lagen, mit einer Träger-Lage, auf welcher ein Kleber angeordnet ist und einer Release-Folien-Lage, zum Ersatz eines Aufbaues aus einer sogenannten Backsheet-Folie, einem Kleber und einer Release-Abdeckbahn, **dadurch gekennzeichnet, dass** wenigstens ein Teil der Lagen coextrudiert ist.

2. Laminat nach Anspruch 1, **dadurch gekennzeichnet, dass** wenigstens die Release-Folien-Lage coextrudiert ist.

3. Laminat nach Anspruch 1 oder 2, **dadurch gekennzeichnet, dass** der Kleber auf eine Träger-Lage aufextrudiert ist, wobei bei dieser Extrusion die ebenfalls coextrudierte Release-Folien-Lage zuläuft.

4. Laminat nach Anspruch 3, **dadurch gekennzeichnet, dass** die Träger-Lage für den Kleber aus einem Folien/Vlies-Verbund besteht.

5. Laminat nach einem der vorhergehenden Ansprüche, **dadurch gekennzeichnet, dass** die Folie der Träger-Lage des Klebers einen hohen Anteil an Füllstoffes, wie z.B. CaC03 enthält.

6. Laminat nach einem der vorhergehenden Ansprüche, **dadurch gekennzeichnet, dass** wenigstens Teile des Laminates gereckt sind.

7. Laminat nach einem der vorhergehenden Ansprüche, **dadurch gekennzeichnet, dass** das Laminat wenigstens einseitig geprägt ist.
